# EUROPEAN PATENT SPECIFICATION

(11) **EP 1 221 950 B1**
(45) Date of publication and mention of the grant of the patent: **16.06.2004**
(21) Application number: 00966138.0
(22) Date of filing: 05.10.2000
(51) Int. Cl.: A61K 31/195, A61K 31/404, A61P 25/02, A61P 25/04, A61K 31/40, A61K 45/06

(54) **SYNERGISTIC COMBINATIONS OF AN NK1 RECEPTOR ANTAGONIST AND A GABA STRUCTURAL ANALOG**
SYNERGISTISCHE KOMBINATION VON NK1-REZEPTOR ANTAGONISTEN UND GABA ANALOGEN
COMPLEXES MEDICAMENTEUX D'ANTAGONISTE DE RECEPTEUR NK1 ET D'ANALOGUE STRUCTUREL GABA

(30) Priority: 07.10.1999 US 158271 P
(43) Date of publication of application: 17.07.2002
(73) Proprietor: Warner-Lambert Company LLC, Morris Plains, New Jersey 07950 (US)
(72) Inventor: HUGHES, John, Swaffham Bulbeck, Cambridgeshire CB5 ONF (GB); SINGH, Lakhbir, Ely, Cambridgeshire CB7 3SP (GB)
(74) Representative: Stuart, Peter Graham
(86) International application number: PCT/EP2000/009858
(87) International publication number: WO 2001/024792

(56) References cited:
- FIELD M J ET AL: "Involvement of the central tachykinin NK1 receptor during maintenance of mechanical hypersensitivity induced by diabetes in the rat" JOURNAL OF PHARMACOLOGY AND EXPERIMENTAL THERAPEUTICS, vol. 285, no. 3, 1998, pages 1226-1232, XP000973157 cited in the application
- MCLEAN S ET AL: "CP-99,994, a nonpeptide antagonist of the tachykinin NK1 receptor" REGULATORY PEPTIDES, vol. 46, no. 1-2, 1993, pages 329-331, XP000972928
- BRYANS JUSTIN S ET AL: "3-substituted GABA analogs with central nervous system activity: A review." MEDICINAL RESEARCH REVIEWS, vol. 19, no. 2, March 1999 (1999-03), pages 149-177, XP000972932 ISSN: 0198-6325
- FIELD M J ET AL: "Gabapentin and the NK1 receptor antagonist CI-1021 act synergistically to block allodynia induced in a rat model of neuropathic pain." BRITISH JOURNAL OF PHARMACOLOGY, vol. 129, no. Proceedings Supplement, January 2000 (2000-01), page 79P XP000972934 Meeting of the British Pharmacological Society; Cambridge, England, UK; January 05-07, 2000 ISSN: 0007-1188

## Description

### FIELD OF THE INVENTION

This invention relates to the use of a combination of a GABA analog and a NK₁ receptor antagonist for preventing or treating chronic pain, and to pharmaceutical compositions of said combination.

### BACKGROUND OF THE INVENTION

Neurokinin 1 (NK₁) receptor antagonists are being developed for the treatment of a number of physiological disorders associated with an excess or imbalance of tachykinins, and in particular of substance P, an important neurotransmitter in the transmission of pain.

A selective NK₁ receptor antagonist, [2-(1*H*-indol-3-yl)-1-methyl-1-(1-phenylethylcarbamoyl)-ethyl]-carbamic acid benzofuran-2-ylmethyl ester, has been shown in the rat to block the maintenance of streptozocin-induced static allodynia (Field *et al*., (1998) J. Pharmacol. Exp. Ther. **285**: 1226-1232).

Gabapentin (1-(aminomethyl)cyclohexane acetic acid) is an antiepileptic drug. This γ-aminobutyric acid (GABA) structural analogue has been shown to possess antihyperalgesic actions in animal models of inflammatory and neuropathic pains, and in particular to block the maintenance of streptozocin-induced static allodynia in the rat (Field *et al*., (1997) J. Pharmacol. Exp. Ther. **282**: 1242-1246).

Chronic painful conditions, in various forms, affect a considerable number of people including, according to the World Health Organization, 4 million cancer sufferers who, worldwide, suffer as a result of a lack of suitable care. There are a number of other conditions, such as musculoskeletal or vertebral pain, neurological pain, headaches or vascular pain. Neuropathic pain, a chronic pain condition occurring in the setting of nervous system injury or tissue injury, is characterized by unusual sensory experiences (allodynia, hyperalgesia) and abnormal pain processing in the central and peripheral nervous systems; treatment of neuropathic pain is difficult. Painful diabetic neuropathy is one of the most frequent complications of diabetes in humans, post-herpetic neuralgia develops in 10-30 % of patients after herpes zoster, phantom limb and stump pain is a common sequela of amputation. Chronic pain may also be caused by a trauma, an entrapment neuropathy (e.g. carpal tunnel syndrome), multiple sclerosis or a polyneuropathy associated with AIDS, alcohol, hypothyroidism, or anticancer chemotherapy.
Conventional treatments of pain fall into two categories: 1) nonsteroidal anti-inflammatory drugs (NSAIDs), used to treat mild pain, but whose therapeutic use is limited by GI adverse effects; 2) morphine and related opioids, used to treat moderate to severe pain but whose therapeutic use is limited by undesirable side effects including respiratory depression, tolerance, and abuse potential. However, conventional analgesics, whether opiates or NSAIDs, have limited therapeutic value in the management of chronic pain syndromes. This has led to the use of adjuvant analgesics for the management of these conditions. For example, tricyclic antidepressants are currently the first choice in the treatment of painful diabetic neuropathy (Galer, 1995; James and Page, 1994). However, no agent is fully effective in all patients and undesirable side effects are common (Galer, 1995; James and Page, 1994).
In view of the multiple organs involved in the presentation of cancer, AIDS, multiple sclerosis, or diabetes, requiring multiple drugs, the treatment of the associated chronic pain warrants careful attention to side effects, contraindications and drug interaction.
To this day, a major problem therefore exists, the resoiution of which has been unsatisfactory, for the treatment of chronic pain. In particular, there is a need for drugs that can alleviate chronic pain without undesirable side effects, and for combination therapy with analgesic agents having distinct mechanisms of action, acting in a synergistic manner.

### SUMMARY OF THE INVENTION

It has now been discovered that combination therapy with a NK₁ receptor antagonist and a GABA analog results in dramatic improvement in chronic pain control. When administered together, the NK₁ receptor antagonist and the GABA analog can interact in a synergistic manner to control chronic pain. This unexpected synergy allows a reduction in the dose required of each compound, leading to a reduction in the side effects, and enhancement of the clinical utility of the compounds.

Accordingly, this invention provides the use of a GABA analog, or a pharmaceutically acceptable salt thereof, in the preparation of a medicament, in synergistic combination with a NK₁ receptor antagonist, or a pharmaceutically acceptable salt thereof, for preventing or treating chronic pain.

Preferably, the condition treated is selected from causalgia, neuropathic pain, diabetic neuropathy, post-surgery or traumatic neuropathy, postherpetic neuralgia, peripheral neuropathy, entrapment neuropathy, phantom limb and stump pain, neuropathy caused by alcohol abuse, HIV infection, multiple sclerosis, hypothyroidism or anticancer chemotherapy.

The invention also concerns a pharmaceutical composition comprising synergistic effective amounts of a NK₁ receptor antagonist, or a pharmaceutically acceptable salt thereof, and a GABA analog, or a pharmaceutically acceptable salt thereof, with at least one pharmaceutically acceptable carrier or excipient.

### BRIEF DESCRIPTION OF THE DRAWINGS

- FIGURE 1a.: Effect of [2-(1*H*-indol-3-yl)-1-methyl-1-(1-phenyl-ethylcarbamoyl)-ethyl]-carbamic acid benzofuran-2-ylmethyl ester (CI-1021) on the maintenance of diabetes-induced static allodynia.
- FIGURE 1b.: Effect of gabapentin on the maintenance of diabetes-induced static allodynia.
Results are shown as median paw withdrawal thresholds (PWT) measured up to 4 h post *p.o.* drug (vertical bars represent 1st and 3rd quartiles) in 8-10 animals per group. *: P<0.05 **: P<0.01 ***: P<0.001 significantly different from vehicle treated group at each time point.
- FIGURE 2a.: Dose response 1 h after drug for gabapentin and [2-(1*H*-indol-3-yl)-1-methyl-1-(1-phenyl-ethylcarbamoyl)-ethyl]-carbamic acid benzofuran-2-ylmethyl ester (CI-1021) on the maintenance of diabetes-induced static allodynia.
- FIGURE 2b.: Dose response 1 h after drug for gabapentin and CI-1021 combinations at the fixed dose ratio of 1:1 on the maintenance of diabetes-induced static allodynia.
- FIGURE 2c.: Dose response 1 h after drug for gabapentin and CI-1021 combinations at the fixed dose ratio of 10:1 on the maintenance of diabetes-induced static allodynia.
- FIGURE 2d.: Dose response 1 h after drug for gabapentin and CI-1021 combinations at the fixed dose ratio of 20:1 on the maintenance of diabetes-induced static allodynia.
- FIGURE 2e.: Dose response 1 h after drug for gabapentin and CI-1021 combinations at the fixed dose ratio of 40:1 on the maintenance of diabetes-induced static allodynia.
- FIGURE 2f.: Dose response 1 h after drug for gabapentin and CI-1021 combinations at the fixed dose ratio of 60:1 on the maintenance of diabetes-induced static allodynia.
- FIGURE 3a.: Dose response 1 h after drug for gabapentin and (2-methoxybenzyl)-((2S,3S)-2-phenyl-piperidin-3-yl)-amine on the maintenance of diabetes-induced static allodynia.
- FIGURE 3b.: Dose response 1 h after drug for gabapentin and (2-methoxybenzyl)-((2S,3S)-2-phenyl-piperidin-3-yl)-amine (CP-99994) combinations at the fixed dose ratio of 20:1 on the maintenance of diabetes-induced static allodynia.
- FIGURE 4a.: Effect of gabapentin on the maintenance of CCI-induced static allodynia.
- FIGURE 4b.: Effect of [2-(1*H*-indol-3-yl)-1-methyl-1-(1-phenyl-ethyl carbamoyl)-ethyl]-carbamic acid benzofuran-2-ylmethyl ester (CI-1021) on the maintenance of CCI-induced static allodynia.
- FIGURE 5a.: Dose response 2 h after drug for gabapentin and for [2-(1*H*-indol-3-yl)-1-methyl-1-(1-phenyl-ethylcarbamoyl)-ethyl]-carbamic acid benzofuran-2-ylmethyl ester (CI-1021) on the maintenance of CCI-induced static allodynia.
- FIGURE 5b.: Dose response 2 h after drug for gabapentin and CI-1021 combinations at the fixed dose ratio of 5:1.
- FIGURE 5c.: Dose response 2 h after drug for gabapentin and CI-1021 combinations at the fixed dose ratio of 10:1.
- FIGURE 6.: Dose response 30 min post drug for [2-(1*H*-indol-3-yl)-1-methyl-1-(1-phenyl-ethylcarbamoyl)-ethyl]-carbamic acid benzofuran-2-ylmethyl ester (CI-1021) in the isolation-induced vocalization model of anxiety in the guinea pig pup. Results are shown as mean % reduction ± SEM in the number of calls *vs*. baseline measurements taken before the treatment.
*: P<0.05 *vs* vehicle group; #: P<0.05 *vs* their own vehicles (not included in the graph for clarity); Kruskall-Wallis test followed by Mann-Whitney test.

### DETAILED DESCRIPTION OF THE INVENTION

According to this invention, a NK₁ receptor antagonist is used in combination with a GABA analog to treat chronic pain in patients in need of such treatment. The compounds can be employed individually or can be combined in a single formulation, for example as a tablet, capsule, syrup, solution, as well as controlled release formulations. In a preferred embodiment, the NK₁ receptor antagonist and GABA analog are formulated individually and administered in the same manner that each is normally used clinically, but with reduced amounts of each.
The NK₁ receptor antagonists, such as capsaicin, heretofore used to treat pain or a disorder exhibiting a pain component can be used herein. Specific NK₁ receptor antagonists that can be used herein are disclosed in U.S. Patent Nos. 3,862,114, 3,912,711, 4,472,305, 4,481,139, 4,680,283, 4,839,465, 5,102,667, 5,162,339, 5,164,372, 5,166,136, 5,232,929, 5,242,944, 5,300,648, 5,310,743, 5,338,845, 5,340,822, 5,378,803, 5,410,019, 5,411,971, 5,420,297, 5,422,354, 5,446,052, 5,451,586, 5,525,712, 5,527,811, 5,536,737, 5,541,195, 5,594,022, 5,561,113, 5,576,317, 5,604,247, 5,624,950, and 5,635,510; World Patent Application Nos. WO 90/05525, WO 91/09844, WO 91/12266, WO 92/06079, WO 92/12151, WO 92/15585, WO 92/20661, WO 92/20676, WO 92/21677, WO 92/22569, WO 93/00330, WO 93/00331, WO 93/01159, WO 93/01160, WO 93/01165, WO 93/01169, WO 93/01170, WO 93/06099, WO 93/10073, WO 93/14084, WO 93/19064, WO 93/21155, WO 94/04496, WO 94/08997, WO 94/29309, WO 95/11895, WO 95/14017; WO 97/19942, WO 97/24356; WO 97/38692, WO 98/02158, and WO 98/07694; European Patent Application Nos. 284942, 327009, 333174, 336230, 360390, 394989, 428434, 429366, 443132, 446706, 484719, 499313, 512901, 512902, 514273, 514275, 515240, 520555, 522808, 528495, 532456, and 591040. Preferred NK₁ receptor antagonists that can be used herein are disclosed in U.S. Patent No. 5,594,022; among these, a more preferred NK₁ receptor antagonist is [2-(1*H*-indol-3-yl)-1-methyl-1-(1-phenyl-ethylcarbamoyl)-ethyl]-carbamic acid benzofuran-2-ylmethyl ester.
The GABA analogs heretofore used to treat pain or a disorder exhibiting a pain component can be used herein. Specific GABA analogs that can be used herein are disclosed in U.S. Patent Nos. 4,024,175 and 5,563,175. Preferred GABA analogs include a cyclic amino acid compound of Formula I: 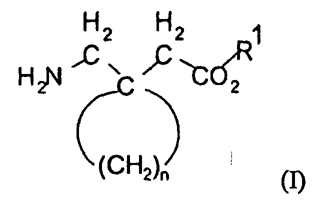 wherein R¹ is hydrogen or lower alkyl and n is an integer of from 4 to 6, and the pharmaceutically acceptable salts thereof. An especially preferred embodiment utilizes a GABA analog of Formula I where R¹ is hydrogen and n is 5, which compound is generically known as gabapentin. Other preferred GABA analogs have Formula I wherein the cyclic ring is substituted, for example with alkyl such as methyl or ethyl. Typical of such compounds include (1-aminomethyl-3-methylcyclohexyl) acetic acid, (1-aminomethyl-3-methylcyclopentyl) acetic acid and (1-aminomethyl-3-4-dimethylcyclopentyl) acetic acid.

In another embodiment, the use of the invention utilizes as a GABA analog a compound of Formula II: 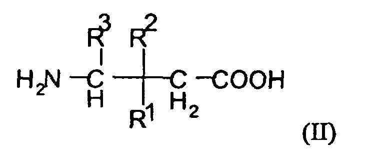 or a pharmaceutically acceptable salt thereof, wherein
R¹ is straight or branched alkyl of from 1 to 6 carbon atoms, phenyl, or cycloalkyl of from 3 to 6 carbon atoms;
R² is hydrogen or methyl; and
R³ is hydrogen, methyl, or carboxyl. Diastereoisomers and enantiomers of compounds of Formula II can be utilized in the invention. An especially preferred use of the invention employs as GABA analog a compound of Formula II where R² and
R³ are both hydrogen, and R¹ is -(CH₂)₀₋₂-*i*C₄H₉ as an (R), (S), or (R,S) isomer. A more preferred embodiment of the invention employs, as GABA analog, 3-aminomethyl-5-methyl-hexanoic acid, and especially (S)-3-aminomethyl-5-methylhexanoic acid, known generically as pregabalin. Another preferred compound of Formula II is 3-(1-aminoethyl)-5-methyl-heptanoic acid.

The dosage of each agent will vary depending upon the severity of the disease, the frequency of administration, the particular agents, and combinations utilized, and other factors routinely considered by an attending medical practitioner. The NK₁ receptor antagonist is normally administered at a daily dose of from about 0.25 mg to about 500 mg, typically about 3 mg to about 250 mg. The GABA analog is normally administered at doses from about 5 mg to about 2500 mg per day, and more typically from about 50 mg to about 1500 mg per day. A preferred GABA analog is gabapentin, and it is employed at doses from about 100 mg to about 1000 mg per day.
A NK₁ receptor antagonist utilized in the present invention includes solvates, hydrates, pharmaceutically acceptable salts, and polymorphs (different crystalline lattice descriptors) of the NK₁ receptor antagonist.
A GABA analog utilized in the present invention includes solvates, hydrates, pharmaceutically acceptable salts, and polymorphs (different crystalline lattice descriptors) of the GABA analog.

Where it is appropriate to form a salt of the NK₁ receptor antagonist or of the GABA analog, the pharmaceutically acceptable salts include acetate, benzenesulfonate, benzoate, bitartrate, calcium acetate, camsylate, carbonate, citrate, edetate, edisylate, estolate, esylate, fumarate, gluceptate, gluconate, glutamate, glycoloylarsanilate, hexylresorcinate, hydrabamine, hydrobromide, hydrochloride, hydrogencarbonate, hydroxynaphthoate, iodide, isethionate, lactate, lactobionate, malate, maleate, mandelate, mesylate, methylnitrate, methylsulfate, mucate, napsylate, nitrate, pamoate (embonate), pantothenate, phosphate/diphosphate, polygalacturonate, salicylate, stearate, subacetate, succinate or hemi-succinate, sulfate or hemi-sulfate, tannate, tartrate or hemi-tartrate, theoclate, triethiodide, benzathine, chloroprocaine, choline, diethanolamine, ethylenediamine, meglumine, procaine, aluminum, ammonium, tetramethyl ammonium, calcium, lithium, magnesium, potassium, sodium, and zinc. (See also "Pharmaceutical salts" by Berge S.M. *et al*. (1997) J. Pharm. Sci. **66**: 1-19.)

The terms "patient" and "subject" are intended to include a mammal, especially a human.

All that is required to practice the use according to the present invention is to administer a synergistic NK₁-GABA analog combination in an amount that is effective to prevent or treat the damaged condition, i.e. to control chronic pain.

In a further aspect of the present invention, there is provided a pharmaceutical composition for the treatment or prevention of chronic pain comprising the synergistic NK₁ antagonist - GABA analog combination. Formulating the active components of the combination in dosage unit form with at least one pharmaceutically acceptable carrier or excipient produces pharmaceutical formulations of the composition according to the present invention. For preparing pharmaceutical formulations from the compounds used in this invention, inert, pharmaceutically acceptable carriers can be either solid or liquid.

Solid dosage forms for oral administration include capsules, tablets, pills, powders, and granules. They preferably contain 5% to about 70% of the active components of the combination. In such solid dosage forms, the active components are admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, carboxymethylcellulose, alginates, gelatin, polyvinylpyrrolidone, sucrose, and acacia, (c) humectants, as for example, glycerol, (d) disintegrating agents, as for example, agar-agar, calcium carbonate, potato or tapioca starch, alginic acid, certain complex silicates, and sodium carbonate, (e) solution retarders, as for example, paraffin, (f) absorption accelerators, as for example, quaternary ammonium compounds, (g) wetting agents, as for example, cetyl alcohol, and glycerol monostearate, (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid compositions of a similar type may also be employed as fillers in soft and hard-filled gelatin capsules using such excipients as lactose as well as high molecular weight polyethyleneglycols, and the like.
Solid dosage forms such as tablets, dragées, capsules, pills, and granules can be prepared with coatings and shells, such as enteric coatings and others well known in the art. They can also be of such composition that they release the active components in a certain part of the intestinal tract in a delayed manner. Examples of embedding compositions that can be used are polymeric substances and waxes. The active components of the combination can also be in microencapsulated form, if appropriate, with one or more of the above-mentioned excipients.
Liquid dosage forms for oral administration include pharmaceutically acceptable emulsions, solutions, suspensions, syrups, and elixirs. In addition to the active components of the combination, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, and the like.
Suspensions, in addition to the active components, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.
Compositions for rectal administrations are preferably suppositories which can be prepared by mixing the active components of the combination of the present invention with suitable non-irritating excipients or carriers such as cocoa butter, polyethyleneglycol or a suppository wax, which are solid at ordinary temperatures but liquid at body temperature, and therefore melt in the rectum and release the active components of the combination.
Compositions suitable for parenteral injection may comprise physiologically acceptable sterile aqueous or nonaqueous solutions, dispersions, suspensions or emulsions of the active components of the combination, and also sterile powders for reconstitution into sterile injectable solutions or dispersions. Examples of suitable liquid carriers, diluents, solvents or vehicles include water, ethanol, polyols (propyleneglycol, polyethyleneglycol, glycerol, and the like), and suitable mixtures thereof.
These compositions may also contain adjuvants such as preserving, wetting, emulsifying, and dispersing agents. Various antibacterial and antifungal agents, for example, parabens, chlorobutanol, phenol, sorbic acid, and the like can ensure prevention of the action of microorganisms. It may also be desirable to include isotonic agents, for example sugars, sodium chloride, and the like.
Preferably the pharmaceutical preparation is in unit dosage form. In such form, the preparation is divided into unit doses containing appropriate quantities of the active components of the combination. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms. Some examples of dosage unit forms are tablets, capsules, pills, powders, suppositories, aqueous and nonaqueous oral solutions and suspensions, and parenteral solutions packaged in containers containing either one or some larger number of dosage units and capable of being subdivided into individual doses.
The percentage of the active components in the foregoing compositions can be varied within wide limits, but for practical purposes it is preferably present in a concentration of at least 10 % in a solid composition and at least 2 % in a primary liquid composition. The most satisfactory compositions are those in which a much higher proportion of the active components is present, for example, from 10 % to 90 % by weight.
Routes of administration of the active components of the combination or their respective salts are parenteral or, preferably, oral. For example, a useful oral dosage is between 20 and 800 mg , expressed as the mass of the GABA analog, and a useful intravenous dose is between 5 and 50 mg. The dosage is within the dosing range used in treatment of pain, or as would be dictated by the needs of the patient as described by the physician.

The invention provides compositions of a NK₁ receptor antagonist and a GABA analog, and the use of a synergistic combination of a NK₁ receptor antagonist and a GABA analog in the preparation of a medicament for the treatment or prevention of chronic pain. Any NK₁ receptor antagonist can be combined with any GABA analog according to this invention. Preferred GABA analogs to be employed are the compounds of Formula I and II, especially gabapentin and pregabalin. Preferred NK₁ receptor antagonists to be employed in the compositions include (2-methoxy-benzyl)-((2S,3S)-2-phenyl-piperidin-3-yl)-amine, and [2-(1*H*-indol-3-yl)-1-methyl-1-(1-phenyl-ethylcarbamoyl)-ethyl]-carbamic acid benzofuran-2-ylmethyl ester.

When the GABA analog and the NK₁ receptor antagonist are formulated together, the composition contains about 1 to about 1000 parts by weight of GABA analog, and about 1000 to about 1 part by weight NK₁ receptor antagonist. Preferred ranges for the ratio of the two active principles, expressed as parts by weight of the GABA analog relative to parts of the NK₁ receptor antagonist, are 50:1 to 1:1. A most preferred range for the ratio of the two active principles is 20:1. For example a typical composition of gabapentin and [2-(1*H*-indol-3-yl)-1-methyl-1-(1-phenylethylcarbamoyl)-ethyl]-carbamic acid benzofuran-2-ylmethyl ester contains about 400 mg of gabapentin and about 20 mg of the NK₁ receptor antagonist. Such combination is administered to an adult patient about twice a day to achieve a synergistic control of chronic pain. The compositions may contain common pharmaceutical excipients such as those described above.

The advantages of using the combination of a NK₁ receptor antagonist and a GABA analog of the instant invention include the selective activity of the combination on chronic pain, the relatively nontoxic nature of the combination, the ease of preparation, the fact that the combination is well tolerated, and the ease of i.v. and, in particular, oral administration of the combination.

The ability of synergistic NK₁-receptor antagonist-GABA analog combinations to prevent or treat chronic pain has been established in several animal models.

### EXAMPLE 1

### Synergistic interaction between a NK₁-receptor antagonist and a GABA analog in animal models of chronic pain

Streptozocin is a selective pancreatic β-cell toxin, which has been used to induce experimental diabetes in laboratory animals (Tomlinson K.C. *et al*., 1992, Pharmacol Rev **44**: 103-150). The resultant loss of endogenous insulin induced by streptozocin mimics the characteristics of type I, or insulin-dependent, diabetes. Streptozocin-induced diabetes has recently been described as a model of chronic pain in rats. It has been reported that streptozocin administration leads to mechanical, thermal, and chemical hyperalgesia- as well as mechanical hypersensitivity (Courteix C. *et al*., 1993, Pain **53**: 81-88; Calcutt N.A. *et al*., 1996, Pain **68**: 293-299). The most common symptoms of diabetic neuropathy appear to be spontaneous burning pain and mechanical hypersensitivity in the feet or lower limbs.
The nerve damage induced by chronic constriction injury (CCI) of the sciatic nerve in the rat produces symptoms similar to those displayed in the clinic, mimicking an entrapment neuropathy, such as the carpal tunnel syndrome, with symptoms also evocative of spontaneous pain, like causalgia. An increased sensitivity to a noxious stimulus (hyperalgesia) develops, together with abnormal pain sensations from previously innocuous stimuli (allodynia) (Bennett G.J. and Xie Y.-K., 1988, Pain **33**: 87-107; Kim S.H. and Chung J.M.,1992, Pain **50**: 355-363).

### Methods:

Male Sprague Dawley rats were housed in groups of 6. All animals were kept under a 12-h light/dark cycle (lights on at 07:00 AM) with food and water *ad libitum*. All experiments were carried out by an observer blind to drug treatments.

### Development of diabetes in the rat

Diabetes was induced in rats (250-300 g) by a single *i.p.* injection of streptozocin (50 mg/kg) as described previously (Courteix *et al*., 1993). Control animals received a similar administration of isotonic saline.

### Induction of chronic constriction injury in the rat

The chronic constriction injury (CCI) was induced as described by Bennett and Xie (1988). Rats were anaesthetized with sodium pentobarbital (60 mg/kg, *i*.*p*.). The common left sciatic nerve was exposed at the level of the middle of the thigh by blunt dissection through *biceps femoris*, and, proximal to the sciatic's trifurcation, four ligatures (4.0 braided silk) were tied loosely around the nerve with about 1 mm spacing. The muscle was closed in layers, and two wound clips were applied to close the skin incision. The nerve damage due to chronic compression develops within 24 h.

### Evaluation of static allodynia

Mechanical hypersensitivity was measured using Semmes-Weinstein von Frey hairs. Animals were placed into wire mesh bottom cages allowing access to the underside of their paws. Animals were habituated to this environment prior to the start of the experiment. Mechanical hypersensitivity was tested by touching the plantar surface of the animals right hind paw with von Frey hairs in ascending order of force (0.7, 1.2, 1.5, 2, 3.6, 5.5, 8.5, 11.8, 15.1 and 29 g) for up to 6 s. Once a withdrawal response was established, the paw was re-tested, starting with the next descending von Frey hair until no response occurred. The highest force of 29 g lifted the paw as well as eliciting a response, thus represented the cut off point. The paw withdrawal threshold (PWT) was defined as the lowest force (in grams) required to elicit a response.

### Drugs used

[2-(1*H*-indol-3-yl)-1-methyl-1-(1-phenyl-ethylcarbamoyl)-ethyl]-carbamic acid benzofuran-2-ylmethyl ester [R-(R*,S*)], was dissolved in saturated polyglycolysed glycerides (Gelucire™) at a temperature of 65 °C and, once dissolved, maintained at 45 °C. Gabapentin, which is 1-(aminomethyl)cyclohexane acetic acid, and (2-methoxy-benzyl)-((2S,3S)-2-phenyl-piperidin-3-yl)-amine were dissolved in water. Streptozocin was dissolved in 0.9 % w/v NaCl aqueous solution. Drugs were administered under a volume of 1 ml/kg.

### Results:

### Synergistic interaction of gabapentin and [2-(1H-indol-3-yl)-1-methyl-1-(1-phenyl-ethylcarbamoyl)-ethyl]-carbamic acid benzofuran-2-ylmethyl ester on streptozocin-induced static allodynia

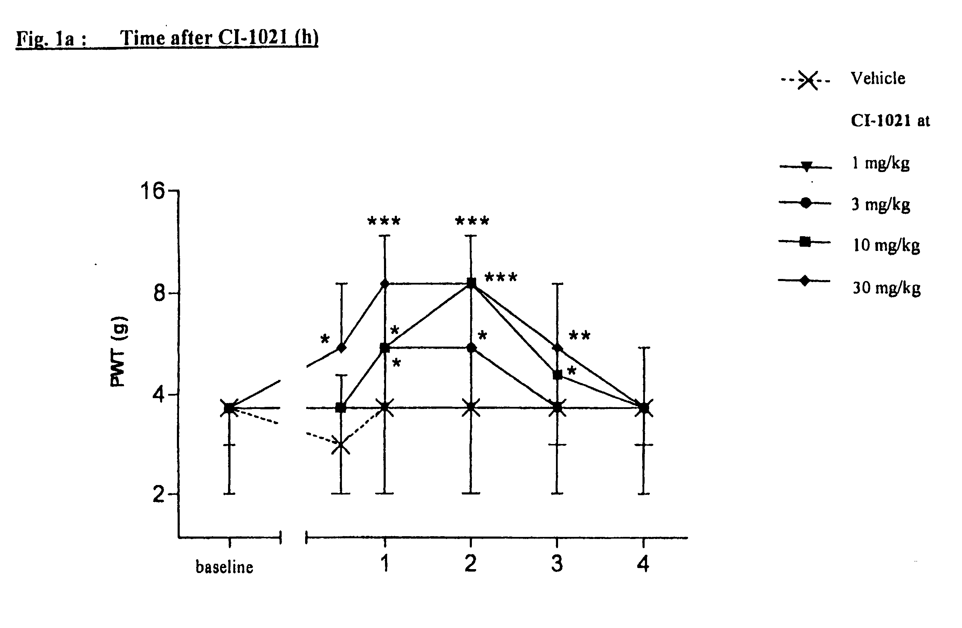

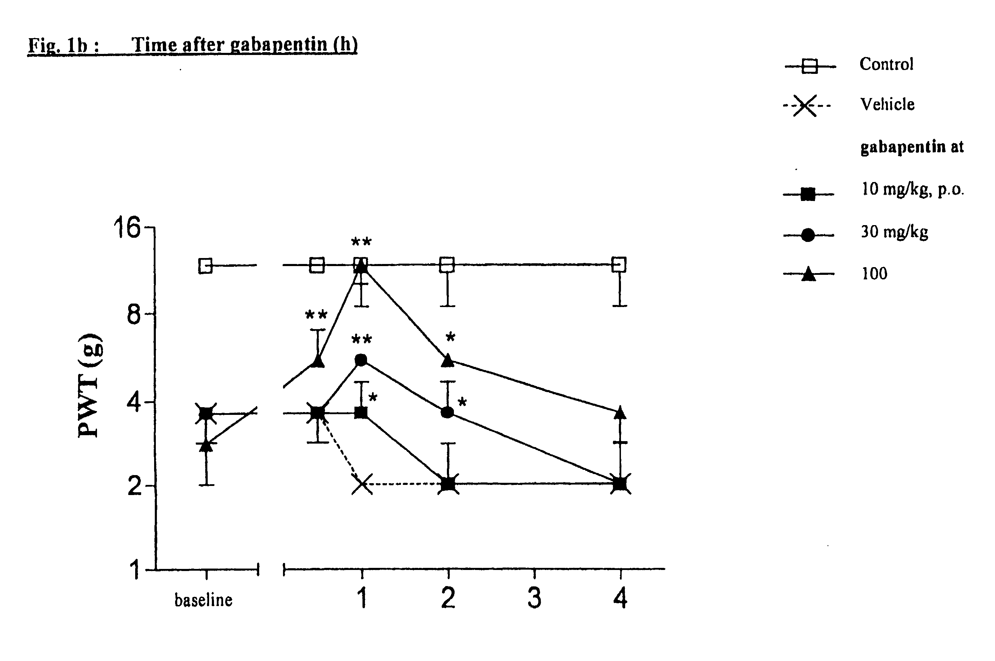

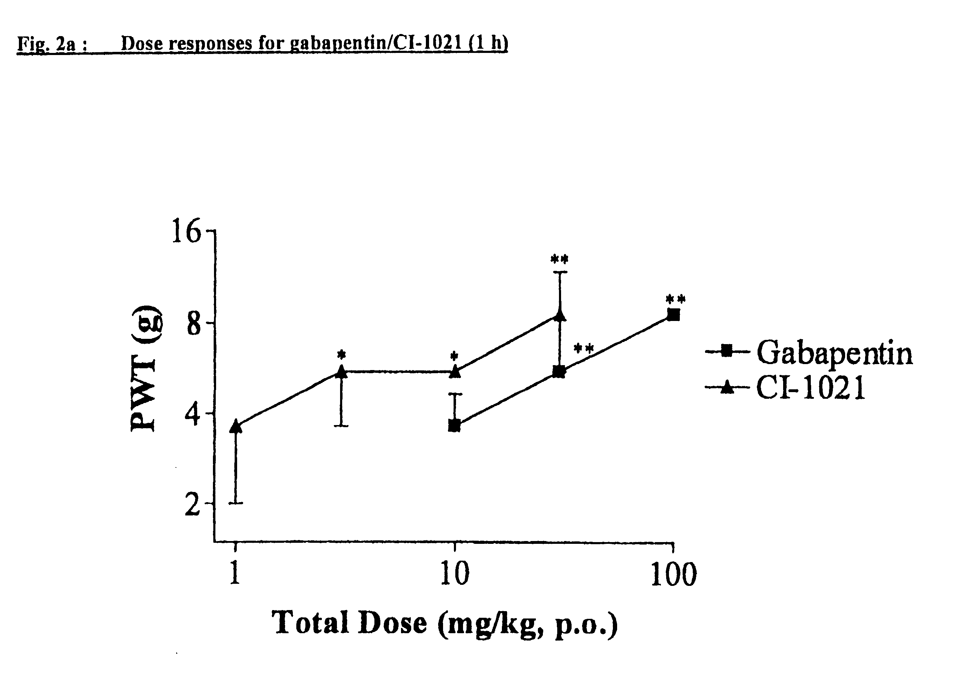

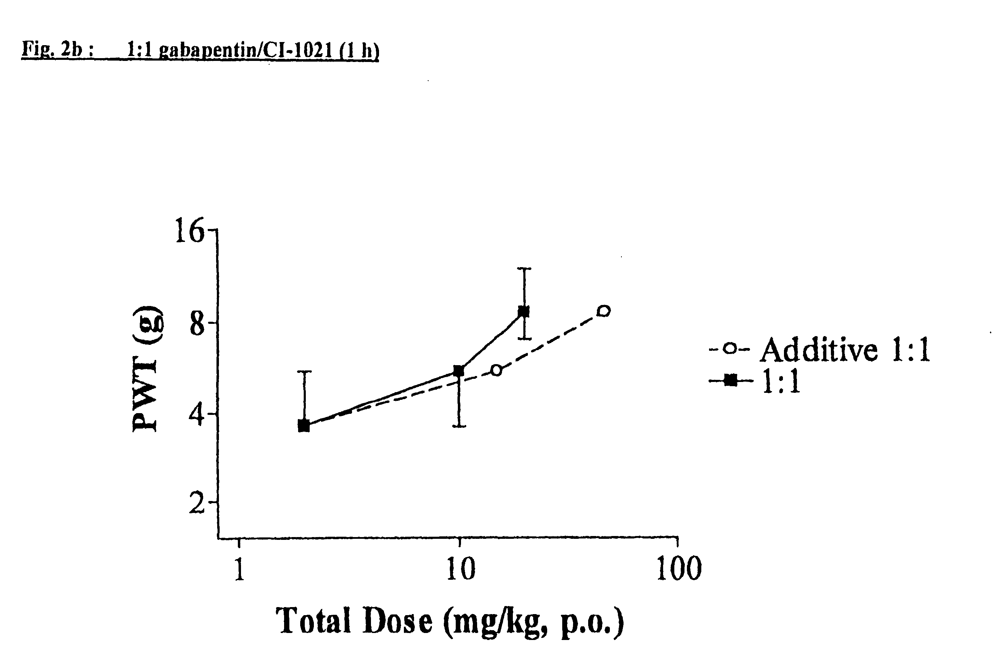

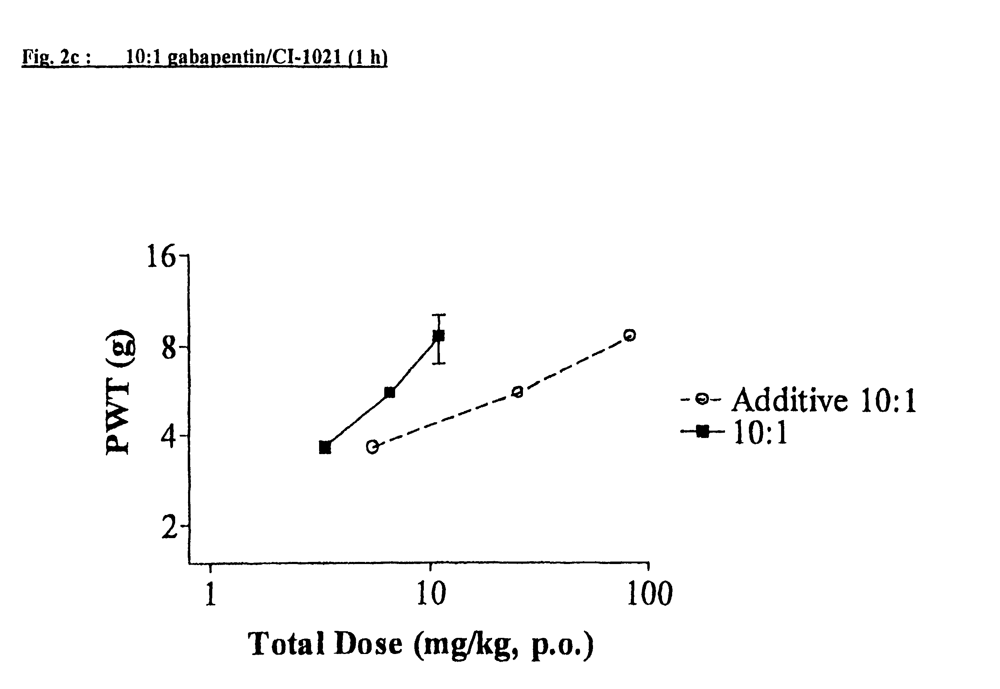

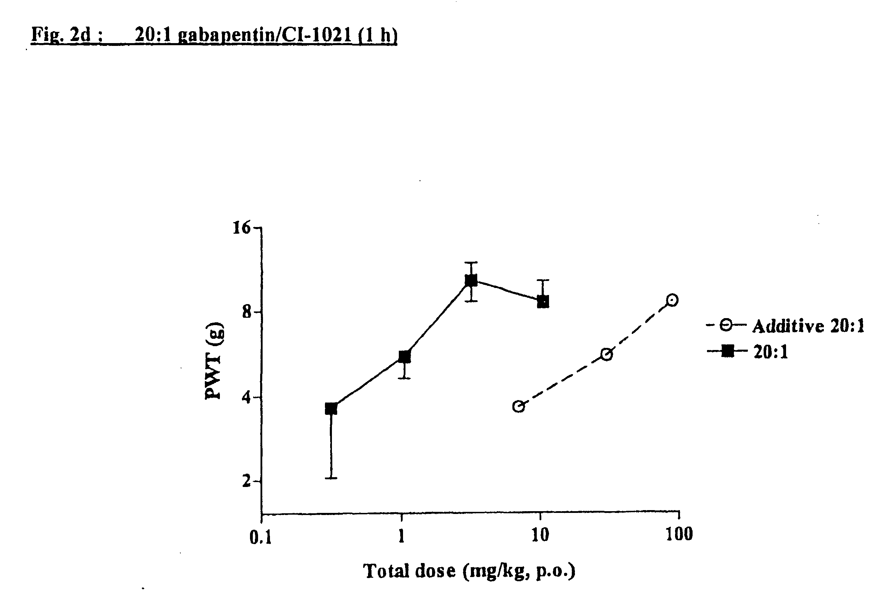

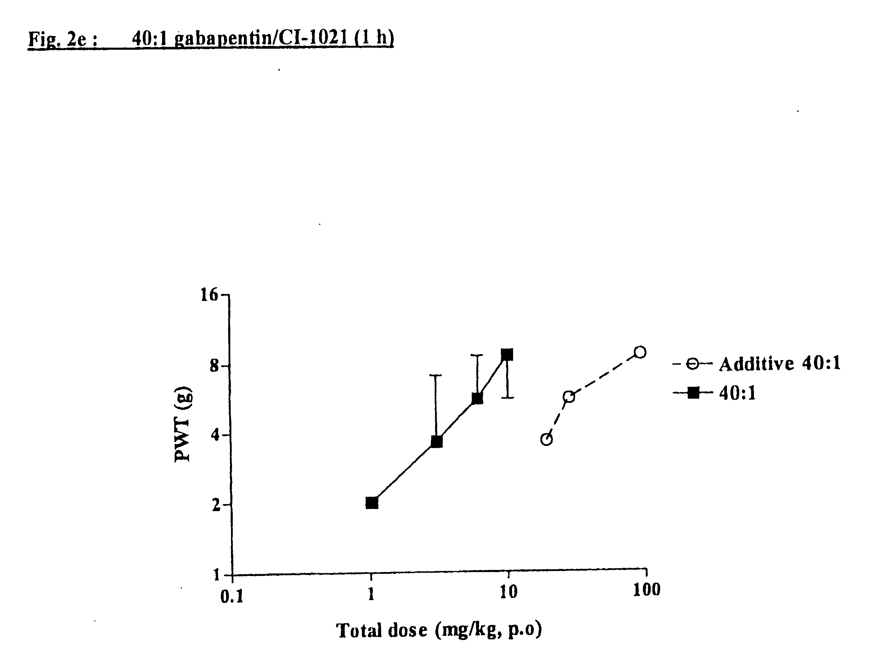

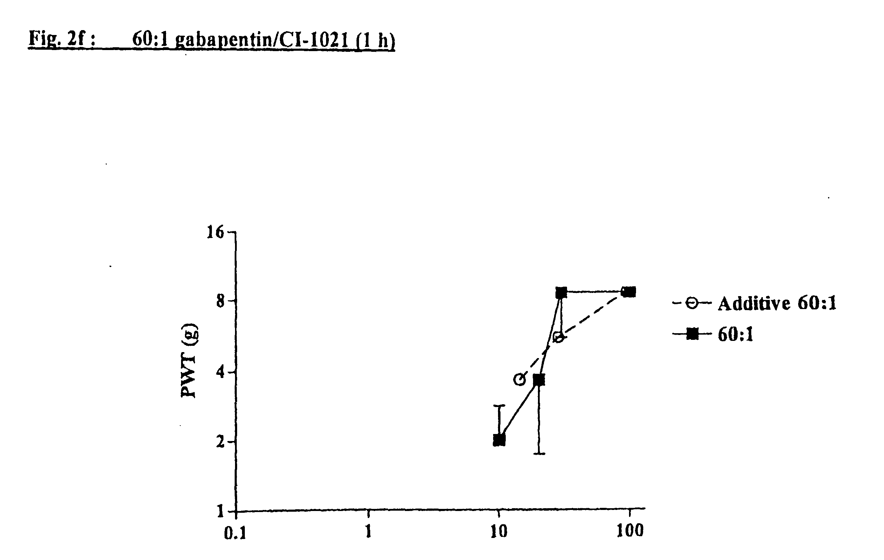

Oral administration 1 h before test of either [2-(1*H*-indol-3-yl)-1-methyl-1-(1-phenyl-ethylcarbamoyl)-ethyl]-carbamic acid benzofuran-2-ylmethyl ester (1 - 30 mg/kg) (Figure 1a) or gabapentin (10 - 100 mg/kg) (Figure 1b) dose-dependently blocked the maintenance of static allodynia, with respective minimum effective doses (MED) of 3 and 10 mg/kg.
[2-(1*H*-indol-3-yl)-1-methyl-1-(1-phenyl-ethylcarbamoyl)-ethyl]-carbamic acid benzofuran-2-ylmethyl ester and gabapentin were administered at fixed dose ratios of 1:1, 10:1, 20:1, 40:1, and 60:1. The dose response data for both compounds were used to determine theoretical additive lines (Figures 2b to 2f), as described by Berenbaum M.C., 1989, Pharmacol Rev **41**: 93-141. Due to the different vehicle used, [2-(1*H*-indol-3-yl)-1-methyl-1-(1-phenyl-ethylcarbamoyl)-ethyl]-carbamic acid benzofuran-2-ylmethyl ester was administered directly after gabapentin. At a fixed dose ratio of 1:1, combinations of [2-(1*H*-indol-3-yl)-1-methyl-1-(1-phenyl-ethylcarbamoyl)-ethyl]-carbamic acid benzofuran-2-ylmethyl ester and gabapentin blocked the development of static allodynia, with a complete blockade at the dose of 10:10 mg/kg (Figure 2b). The data was very close to the theoretical additive line indicating an additive interaction (Figure 2b). However, following a fixed dose ratio of 10:1, 20:1 and 40:1, static allodynia was completely blocked by the 10 mg/kg doses. It should be noted that at this dose, neither of the compounds alone showed activity in this model (Figure 2a). The 20:1 ratio was approximately 10 fold more potent than the theoretical additive line (Figure 2d) indicating synergy at this dose ratio.

### Synergistic interaction of gabapentin and (2-methoxy-benzyl)-((2S,3S)-2-phenylpiperidin-3-yl)-amine on streptozocin-induced static allodynia

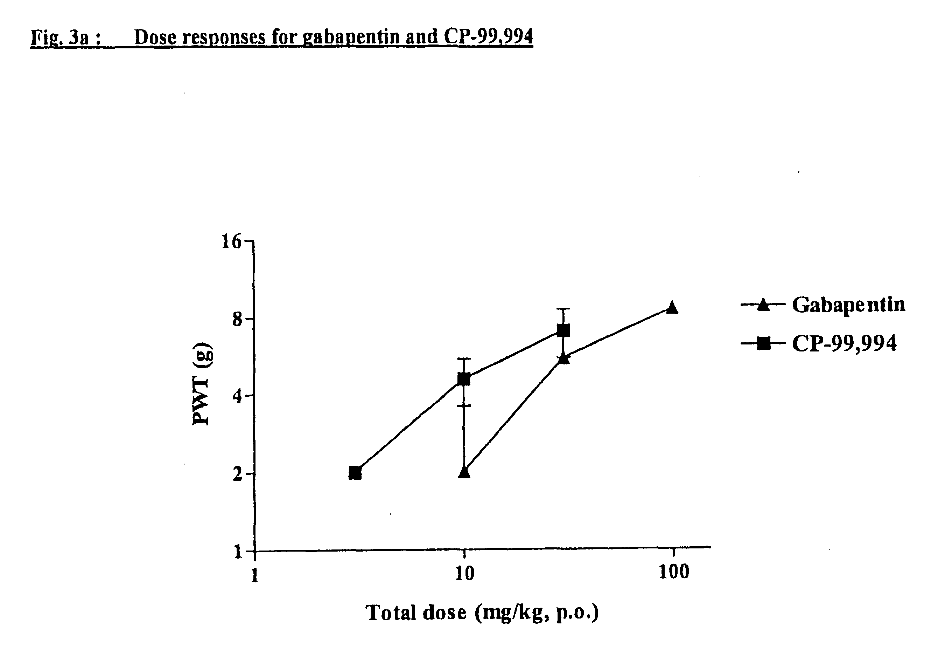

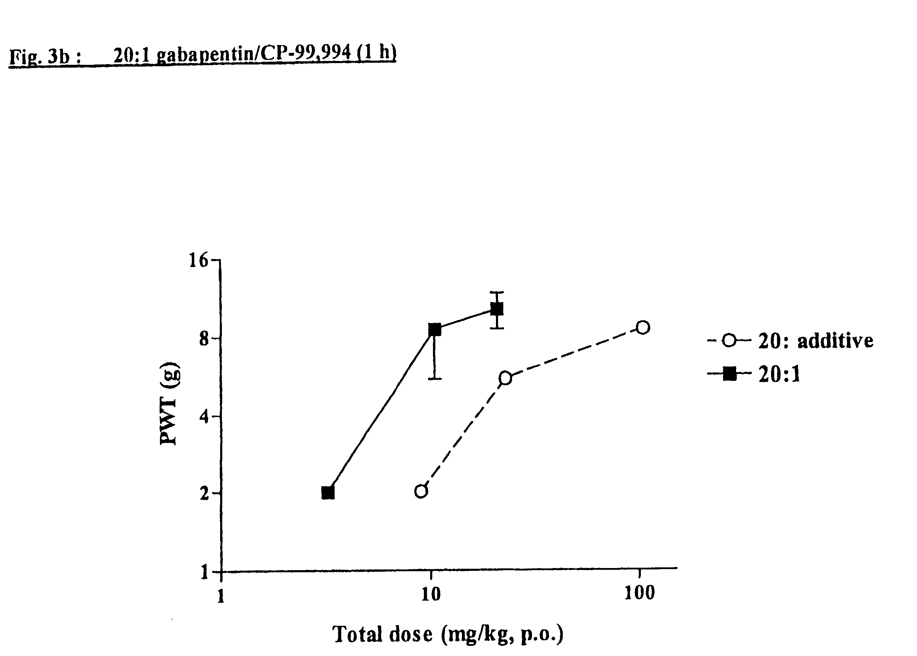

Oral administration 1 h before test of either (2-methoxy-benzyl)-((2S,3S)-2-phenyl-piperidin-3-yl)-amine (3 - 30 mg/kg) or gabapentin (10 - 100 mg/kg) dose-dependently blocked the maintenance of static allodynia (Figure 3a).
(2-Methoxy-benzyl)-((2S,3S)-2-phenyl-piperidin-3-yl)-amine and gabapentin were administered at the fixed dose ratio of 20:1. The dose response data for both compounds were used to determine the theoretical additive line (Figure 3b). In rats given the combination at the fixed dose ratio of 20:1, static allodynia was completely blocked by the 10-mg/kg dose. Again at this dose, neither of the compounds alone showed such activity in this model. The 20:1 ratio was approximately 3 fold more potent than the theoretical additive line, indicating synergy at this dose ratio.

### Synergistic interaction of [2-(1H-indol-3-yl)-1-methyl-1-(1-phenyl-ethylcarbamoyl)-ethyl]-carbamic acid benzofuran-2-ylmethyl ester and gabapentin on CCI-induced static allodynia

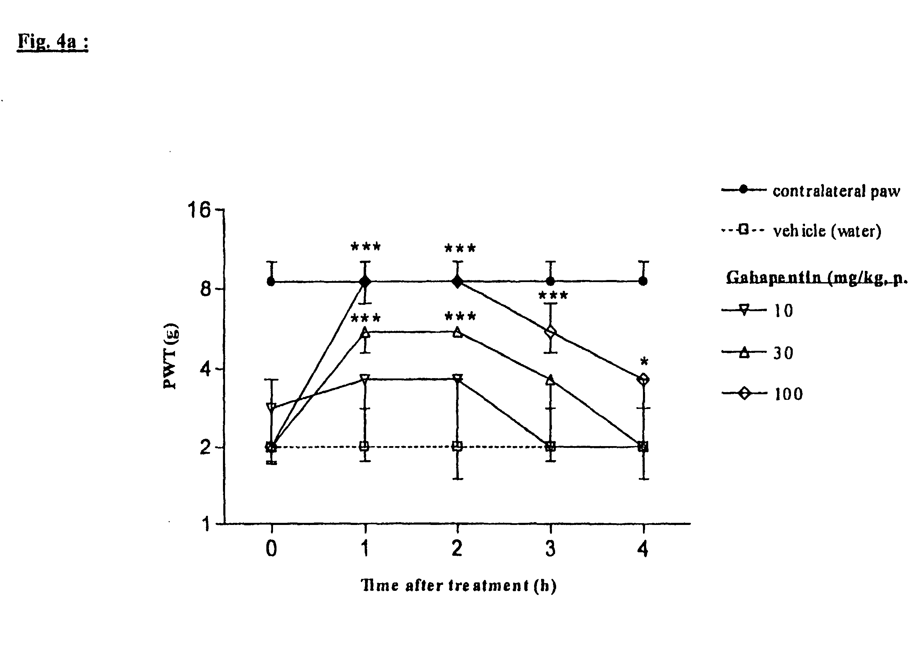

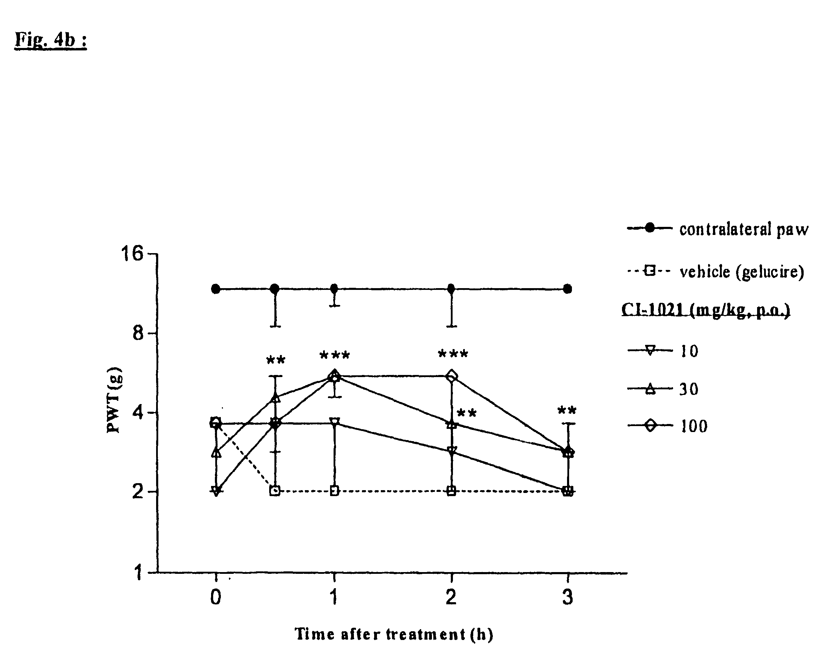

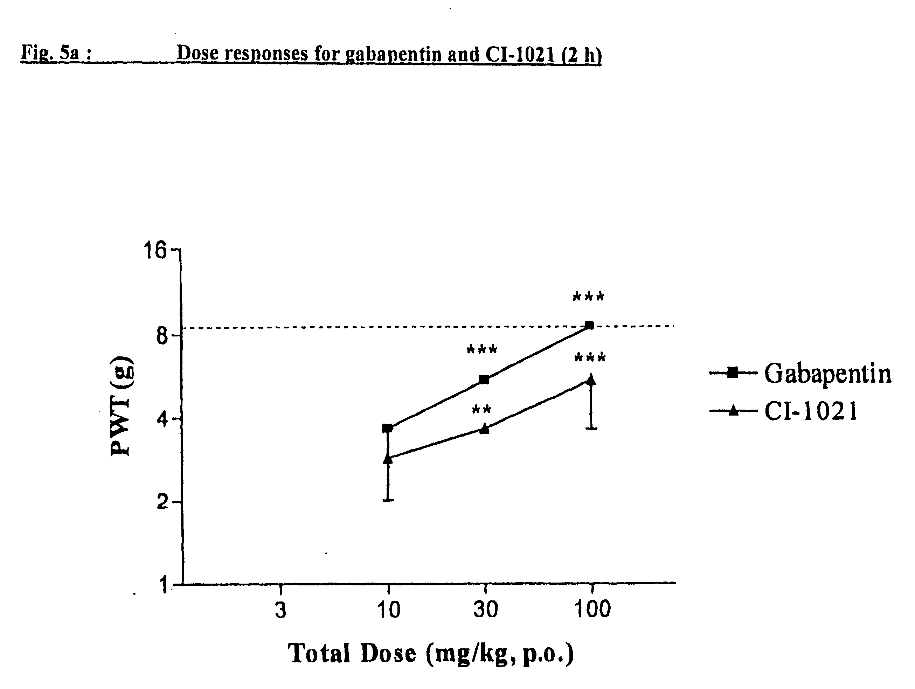

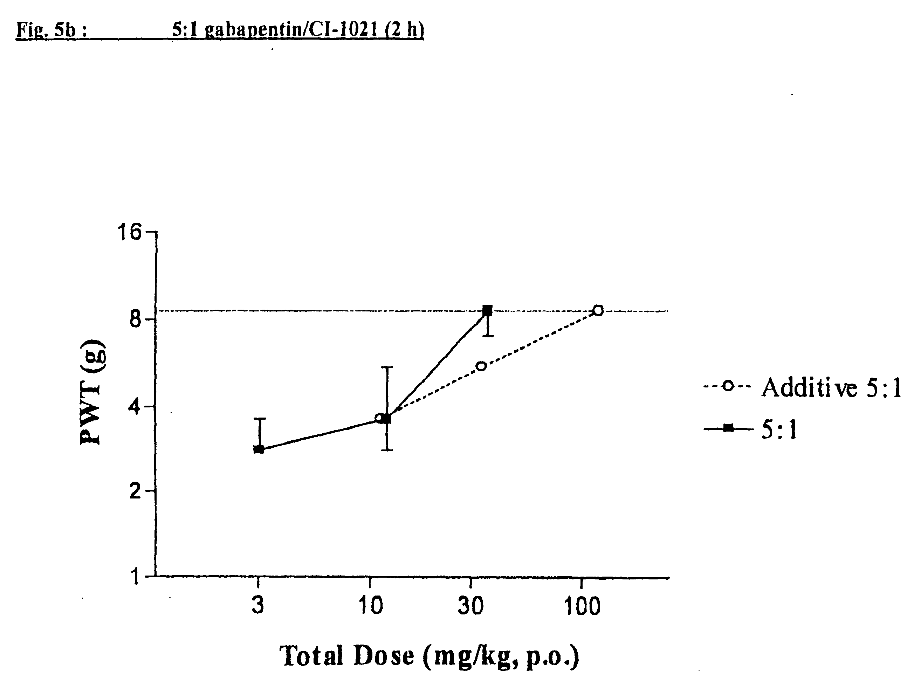

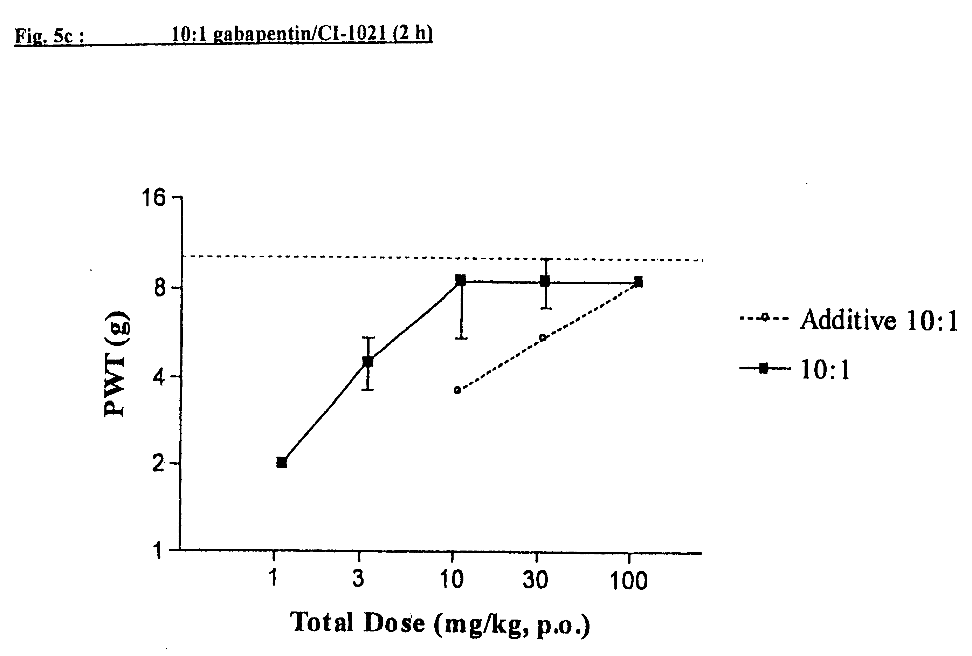

Oral administration (10-100 mg/kg) of either gabapentin (Figure 4a) or [2-(1*H*-indol-3-yl)-1-methyl-1-(1-phenyl-ethylcarbamoyl)-ethyl]-carbamic acid benzofuran-2-ylmethyl ester (Figure 4b) also dose-dependently blocked the maintenance of CCI-induced static allodynia with MEDs of 30 mg/kg. The highest dose (100 mg/kg) of gabapentin, but not of [2-(1*H*-indol-3-yl)-1-methyl-1-(1-phenyl-ethylcarbamoyl)-ethyl)-carbamic acid benzofuran-2-ylmethyl ester, completely blocked static allodynia.
[2-(1*H*-indol-3-yl)-1-methyl-1-(1-phenyl-ethylcarbamoyl)-ethyl]-carbamic acid benzofuran-2-ylmethyl ester and gabapentin were administered at fixed dose ratios of 5:1 and 10:1. The dose response data for both compounds were used to determine theoretical additive lines as described by Berenbaum, *Ibidem* (Figure 5b, 5c). Due to the different vehicle used, [2-(1*H*-indol-3-yl)-1-methyl-1-(1-phenyl-ethylcarbamoyl)-ethyl]-carbamic acid benzofuran-2-ylmethyl ester was administered directly after gabapentin. Two hours after administration as a fixed dose ratio of 5:1, combinations of [2-(1*H*-indol-3-yl)-1-methyl-1-(1-phenyl-ethylcarbamoyl)-ethyl)-carbamic acid benzofuran-2-ylmethyl ester and gabapentin blocked the development of static allodynia, showing a complete blockade at the dose of 30:6 mg/kg (Figure 5b). At this dose level, gabapentin alone shows a significant effect but does not produce total blockade, while [2-(1*H*-indol-3-yl)-1-methyl-1-(1-phenyl-ethylcarbamoyl)-ethyl]-carbamic acid benzofuran-2-ylmethyl ester has no effect (Figure 5a). The 5:1 ratio was approximately 3 fold more potent than the theoretical additive line (Figure 5b) indicating modest synergy at this dose ratio. However, following a fixed dose ratio of 10:1 static allodynia was completely blocked 2 h after administration at the doses 10:1 mg/kg (Figure 5c). It should be noted that at these doses neither of the compounds alone showed activity in this model (Figure 5a). The 10:1 ratio was approximately 10 fold more potent than the theoretical additive line (Figure 5c) indicating synergy at this dose ratio.
Therefore, gabapentin or [2-(1*H*-indol-3-yl)-1-methyl-1-(1-phenyl-ethylcarbamoyl)-ethyl]-carbamic acid benzofuran-2-ylmethyl ester can block the maintenance of streptozocin- and CCI-induced static allodynia when administered alone. However, when administered together at a combination ratio of 10:1, 20:1 or 40:1, the two compounds can interact in a synergistic manner to block pain, in both neuropathic pain models. The synergy also exists between gabapentin and others NK₁ receptor antagonists such as (2-methoxy-benzyl)-((2S,3S)-2-phenylpiperidin-3-yl)-amine. The reduction in dose required of each compound will lead to a reduction in the side effects, and enhance the clinical utility of the compounds.

### EXAMPLE 2

### Synergistic interaction between a NK₁-receptor antagonist and a GABA analog in isolation-induced vocalizations of guinea-pig pups

### Methods:

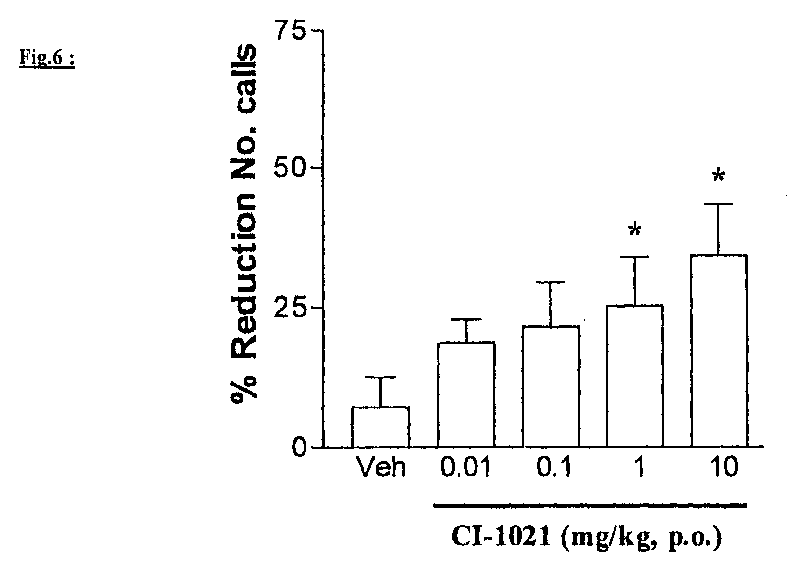

Distress vocalizations of guinea-pig pups (2-14 days old) are quantified in a 5-min isolation period, after which they are reunited with their mothers and littermates. The test cage consists of a sound-attenuating box with a white interior and white illumination. The vocalizations are recorded by means of a microphone and a digital audio tape (DAT) recorder. Pups are first selected using the criterion of emitting a minimum of 500 vocalizations after three pre-tests on three consecutive days. On the day of the test, pups are submitted to a pre-treatment (baseline) measurement. Each pup then receives oral administration of test compounds and is returned to the home cage for 30 min before maternal separation.
Different ratios of combinations of doses are administered to groups of animals (n= 9-10 per group). A minimum of 3 total doses for each ratio of combination is examined. The difference in the number of calls emitted before and after treatment is counted using Spike2 software; percentage of reduction in the number of calls is analyzed using a Kruskall-Wallis test followed by Mann-Whitney test between vehicle and different treatments. For example, the oral administration of [2-(1*H*-indol-3-yl)-1-methyl-1-(1-phenyl-ethylcarbamoyl)-ethyl]-carbamic acid benzofuran-2-ylmethyl ester (0.01-10.0 mg/kg *p.o.,* in Gelucire™ vehicle 30 min before the test) dose-dependently blocked vocalizations with a MED of 1.0 mg/kg (Figure 6). With different ratios of combinations of doses of a NK₁ receptor antagonist and a GABA analog, a synergistic interaction is considered when a significant shift to the left from the additive line is achieved.

The following examples illustrate typical formulations provided by the invention.

### EXAMPLE 3

| Tablet Formulation | |
|---|---|
| Ingredient | Amount (mg) |
| [2-(1H-indol-3-yl)-1-methyl-1-(1-phenyl-ethylcarbamoyl)-ethyl]-carbamic acid benzofuran-2-ylmethyl ester [R-(R*,S*)] | 5 |
| Gabapentin | 100 |
| Lactose | 95 |
| Corn starch (for mix) | 20 |
| Com starch (paste) | 20 |
| Magnesium stearate (1%) | 10 |
| Total | 250 |

The benzofuranyl-methyl ester, gabapentin, lactose, and corn starch (for mix) are blended to uniformity. The corn starch (for paste) is suspended in 400 mL of water and heated with stirring to form a paste. The paste is used to granulate the mixed powders. The wet granules are passed through a No. 8 hand screen and dried. The dry granules are lubricated with the 1 % magnesium stearate and pressed into a tablet. Such tablets can be administered to a human from one to four times a day for treatment of chronic pain.

### EXAMPLE 4

| Preparation for Oral Solution | |
|---|---|
| Ingredient | Amount |
| [2-(1H-indol-3-yl)-1-methyl-1-(1-phenyl-ethylcarbamoyl)-ethyl]-carbamic acid benzofuran-2-ylmethyl ester [R-(R*,S*)] | 20 mg |
| Pregabalin | 400 mg |
| Sorbitol solution (70% N.F.) | 40 mL |
| Sodium benzoate | 20 mg |
| Saccharin | 5 mg |
| Red dye | 10 mg |
| Cherry flavor | 20 mg |
| Distilled water q.s. | 100 mL |

The sorbitol solution is added to 40 mL of distilled water, and pregabalin and the benzofuranylmethyl ester are dissolved therein. The saccharin, sodium benzoate, flavor, and dye are added and dissolved. The volume is adjusted to 100 mL with distilled water.

### EXAMPLE 5

### Parenteral Solution

In a solution of 700 mL of propylene glycol and 200 mL of water for injection is added 0.5 g of [2-(1*H*-indol-3-yl)-1-methyl-1-(1-phenyl-ethylcarbamoyl)-ethyl]-carbamic acid benzofuran-2-ylmethyl ester [R-(R*,S*)] and 10 g of pregabalin. The pH is adjusted to 6.5, and the volume is made up to 1000 mL with water for injection. The formulation is sterilized, filled into 5.0 mL ampoules each containing 2.0 mL, and sealed under nitrogen.

## Claims

1. The use of a GABA analog, or a pharmaceutically acceptable salt thereof, in the preparation of a medicament, in synergistic combination with a NK₁ receptor antagonist, or a pharmaceutically acceptable salt thereof, for preventing or treating chronic pain.

2. The use of a NK₁ receptor antagonist, or a pharmaceutically acceptable salt thereof, in the preparation of a medicament, in synergistic combination with a GABA analog, or a pharmaceutically acceptable salt thereof, for preventing or treating chronic pain.

3. The use according to claim 1 or claim 2 wherein the ratio of the GABA analog relative to the NK₁ receptor antagonist is from 50:1 to 1:1 expressed as parts by weight.

4. The use according to claim 3 wherein the ratio of the GABA analog relative to the NK₁ receptor antagonist is 20:1 expressed as parts by weight.

5. The use according to any of Claims 1-4 wherein the NK₁ receptor antagonist is [2-(1*H*-indol-3-yl)-1-methyl-1-(1-phenyl-ethylcarbamoyl)-ethyl]-carbamic acid benzofuran-2-ylmethyl ester [R-(R*,S*)], or a pharmaceutically acceptable salt thereof.

6. The use according to any one of Claims 1-5, wherein the GABA analog is gabapentin, or a pharmaceutically acceptable salt thereof.

7. The use according to any one of Claims 1-5, wherein the GABA analog is pregabalin, or a pharmaceutically acceptable salt thereof

8. The use according to any one of Claims 1-7 wherein the chronic pain is selected from causalgia, neuropathic pain, diabetic neuropathy, post-surgery or traumatic neuropathy, postherpetic neuralgia, peripheral neuropathy, entrapment neuropathy, phantom limb and stump pain, neuropathy caused by alcohol abuse, HIV infection, multiple sclerosis, hypothyroidism or anticancer chemotherapy.

9. A pharmaceutical composition comprising synergistic effective amounts of a NK₁ receptor antagonist, or a pharmaceutically acceptable salt thereof, and a GABA analog, or a pharmaceutically acceptable salt thereof, with at least one pharmaceutically acceptable carrier or excipient.

## Patentansprüche

1. Verwendung eines GABA-Analogen oder eines pharmazeutisch akzeptablen Salzes hiervon bei der Herstellung eines Medikaments in synergistischer Kombination mit einem NK₁-Rezeptor-Antagonisten oder einem pharmazeutisch akzeptablen Salz hiervon zur Verhinderung oder Behandlung von chronischen Schmerzen.

2. Verwendung eines NK₁-Rezeptor-Antagonisten oder eines pharmazeutisch akzeptablen Salzes hiervon bei der Herstellung eines Medikaments in synergistischer Kombination mit einem GABA-Analogen oder einem pharmazeutisch akzeptablen Salz hiervon zur Verhinderung oder Behandlung von chronischen Schmerzen.

3. Verwendung nach Anspruch 1 oder Anspruch 2, wobei das Verhältnis - ausgedrückt in Gewichtsteilen - des GABA-Analogen zu dem NK₁-Rezeptor-Antagonisten in einem Bereich von 50:1 bis 1:1 liegt.

4. Verwendung nach Anspruch 3, wobei das Verhältnis - ausgedrückt in Gewichtsteilen - des GABA-Analogen zu dem NK₁-Rezeptor-Antagonisten 20:1 beträgt.

5. Verwendung nach einem der Ansprüche 1 bis 4, wobei der NK₁-Rezeptor-Antagonist [R-(R*,S*)]-[2-(1H-Indol-3-yl)-1-methyl-1-(1-phenyl-ethylcarbamoyl)ethyl)]carbaminsäurebenzofuran-2-ylmethylester oder ein pharmazeutisch akzeptables Salz hiervon ist.

6. Verwendung nach einem der Ansprüche 1 bis 5, wobei das GABA-Analoge Gabapentin oder ein pharmazeutisch akzeptables Salz hiervon ist.

7. Verwendung nach einem der Ansprüche 1 bis 5, wobei das GABA-Analoge Pregabalin oder ein pharmazeutisch akzeptables Salz hiervon ist.

8. Verwendung nach einem der Ansprüche 1 bis 7, wobei die chronischen Schmerzen aus Kausalgie, neuropathischen Schmerzen, diabetischer Neuropathie, postoperativer oder traumatischer Neuropathie, Postherpesneuralgie, peripherer Neuropathie, Nervenkompressionssyndrom, Phantomglied- und -stumpfschmerz, Neuropathie verursacht durch Alkoholmissbrauch, HIV-Infektion, Multiple Sklerose, Hyposchilddrüsenunterfunktion oder Antikrebschemotherapie ausgewählt sind.

9. Pharmazeutische Zusammensetzung, die synergistische wirksame Mengen eines NK₁-Rezeptor-Antagonisten oder eines pharmazeutisch akzeptablen Salzes hiervon und eines GABA-Analogen oder eines pharmazeutisch akzeptablen Salzes hiervon zusammen mit mindestens einem pharmazeutisch akzeptablen Träger oder Streckmittel umfasst.

## Revendications

1. Utilisation d'un analogue du GABA, ou d'un sel pharmaceutiquement acceptable de celui-ci, dans la préparation d'un médicament, en combinaison synergique avec un antagoniste du récepteur NK₁, ou un sel pharmaceutiquement acceptable de celui-ci, pour prévenir ou traiter la douleur chronique.

2. Utilisation d'un antagoniste du récepteur NK₁, ou d'un sel pharmaceutiquement acceptable de celui-ci, dans la préparation d'un médicament, en combinaison synergique avec un analogue du GABA, ou un sel pharmaceutiquement acceptable de celui-ci, pour prévenir ou traiter la douleur chronique.

3. Utilisation selon la revendication 1 ou la revendication 2 dans laquelle la proportion de l'analogue du GABA par rapport à l'antagoniste du récepteur NK₁ est de 50:1 à 1:1 exprimée en parties en poids.

4. Utilisation selon la revendication 3 dans laquelle la proportion de l'analogue du GABA par rapport à l'antagoniste du récepteur NK₁ est de 20:1 exprimée en parties en poids.

5. Utilisation selon l'une quelconque des revendications 1-4 dans laquelle l'antagoniste du récepteur NK₁ est l'ester [R-(R*,S*)] benzofuran-2-ylméthylique de l'acide [2-(1*H*-indol-3-yl)-1-méthyl-1-(1-phényl-éthylcarbamoyl)-éthyl]-carbamique, ou un sel pharmaceutiquement acceptable de celui-ci.

6. Utilisation selon l'une quelconque des revendications 1-5, dans laquelle l'analogue du GABA est la gabapentine, ou un sel pharmaceutiquement acceptable de celle-ci.

7. Utilisation selon l'une quelconque des revendications 1-5, dans laquelle l'analogue du GABA est la prégabaline, ou un sel pharmaceutiquement acceptable de celle-ci.

8. Utilisation selon l'une quelconque des revendications 1-7 dans laquelle la douleur chronique est choisie parmi la causalgie, la douleur neuropathique, la neuropathie diabétique, la neuropathie postopératoire ou traumatique, la névralgie post-herpétique, la neuropathie périphérique, les syndromes des défilés, la douleur du membre fantôme et du moignon, la neuropathie causée par un abus d'alcool, l'infection à VIH, la sclérose en plaques, l'hypothyroïdie et la chimiothérapie anticancéreuse.

9. Composition pharmaceutique comprenant des quantités synergiques efficaces d'un antagoniste du récepteur NK₁, ou d'un sel pharmaceutiquement acceptable de celui-ci, et d'un analogue du GABA, ou d'un sel pharmaceutiquement acceptable de celui-ci, avec au moins un vecteur ou excipient pharmaceutiquement acceptable.
